(19) Europäisches Patentamt
European Patent Office
Office européen des brevets

(11) **EP 2 901 928 B1**

(12) **EUROPEAN PATENT SPECIFICATION**

(45) Date of publication and mention
of the grant of the patent:
**31.01.2018 Bulletin 2018/05**

(21) Application number: **13840482.7**

(22) Date of filing: **26.09.2013**

(51) Int Cl.:
**A61B 5/1455** *(2006.01)*　　**A61B 10/00** *(2006.01)*

(86) International application number:
**PCT/JP2013/076013**

(87) International publication number:
**WO 2014/050945 (03.04.2014 Gazette 2014/14)**

(54) **NON-INVASIVE LIVING BODY MEASUREMENT DEVICE**

NICHTINVASIVE MESSVORRICHTUNG FÜR LEBENDKÖRPER

DISPOSITIF DE MESURE DE CORPS VIVANT NON INVASIF

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.09.2012 JP 2012217216**

(43) Date of publication of application:
**05.08.2015 Bulletin 2015/32**

(73) Proprietor: **Sysmex Corporation Kobe-shi, Hyogo 651-0073 (JP)**

(72) Inventors:
* **NISHIMOTO, Naomichi**
  **Tachikawa-shi**
  **Tokyo 190-0004 (JP)**
* **MURAYAMA, Takeo**
  **Tachikawa-shi**
  **Tokyo 190-0004 (JP)**
* **SUZUKI, Tomoaki**
  **Tachikawa-shi**
  **Tokyo 190-0004 (JP)**
* **SAITOU, Takeo**
  **Kobe-shi**
  **Hyogo 651-0073 (JP)**

(74) Representative: **Hoffmann Eitle Patent- und Rechtsanwälte PartmbB Arabellastraße 30 81925 München (DE)**

(56) References cited:
**WO-A1-99/00053　　WO-A1-2009/133851
JP-A- 2004 350 863　　JP-A- 2008 067 727
US-A1- 2002 048 014　　US-A1- 2008 306 366
US-A1- 2009 174 766**

## EP 2 901 928 B1

**Description**

Technical Field

The present invention relates to a non-invasive living body measurement device that irradiates a finger of a subject with light and obtains transmitted light, captures an image of the finger by receiving the transmitted light with an imaging unit, and detects biological information regarding the subject by processing the obtained image.

Background Art

A non-invasive living body measurement device is known in which a finger of a subject is irradiated with light, optical information is acquired by receiving transmitted light or reflected light therefrom with a light receiving unit, and biological information regarding the subject is obtained by analyzing the optical information (e.g., Patent Document 1). The noninvasive living body measurement apparatus disclosed in Patent Document 1 includes a platform on which the finger of the subject is placed, a cover unit that covers the finger placed on the platform, two light source units attached to the cover unit, and an imaging unit. An image of the subject's finger is captured by an imaging unit receiving transmitted light that passes through the finger when light is emitted from the light source units, thus obtaining a transmission image of the subject's finger, and the hemoglobin concentration of the subject is detected by processing the image.

US 2009/174766 A1 describes a finger vein authentication unit including a light source which irradiates infrared light to a finger and an imaging sensor which images a vein image by the light which is diffused in the finger and transmitted through the front side of the finger. The photographing operation is performed in a state in which the first joint is laid on the central portion of a groove. Since the skin of the first joint is thin, the vein can be photographed using a smaller amount of light compared with other portions. Accordingly, strong light is irradiated to the portions other than the first joint and slightly weak light is irradiated to the first joint. Thus, the amount of light of the whole image becomes uniform.

US 2002/048014A1 describes a personal identification system which uses a vein pattern of a finger, optimizes the amount of light of a light source based on a captured finger image and emphasizes the vein pattern during image processing for identification. When human being's finger or toe is imaged, the light transmission factor is highest in joints. Therefore, the system detects a joint from the light intensity profile of a finger in the major axis in the image data and uses the maximum intensity value as the intensity value of the joint.

US 2008/306366A1 describes a biological-information obtaining apparatus including a light-emitting unit configured to emit light, an image-capturing unit configured to capture images, in time sequence, obtained by irradiating a living body with the light emitted and by causing the light to be transmitted through or reflected by the living body. The biological-information obtaining apparatus can be used as a vein authentication apparatus. That is, the use of such a vein authentication apparatus can achieve both identifying of an individual on the basis of vein authentication and obtaining of biological information (information regarding health) regarding the individual.

JP 2008-067727 describes a personal identification device comprising a plurality of light emitting units arranged in a line along a lengthwise direction of a finger, wherein the light emitting units are each equipped with a plurality of light emitting devices configured to emit light of mutually different wavelengths. A difference between finger vein images of different wavelengths is determined to judge whether the finger is a finger of a living body or a forged finger.

Citation List

Patent Documents

Patent Document 1: JP 2000-262496A

Summary of the Invention

Technical Problem

However, with the noninvasive living body measurement device disclosed in Patent Document 1, it has not been possible for the entirety of the subject's finger, which is the measurement target, to be uniformly irradiated with light from the light source units provided on the cover unit. For this reason, obtained images have locally dark and light portions, and have not been clear images.

The present invention has been achieved in light of this situation, and an object thereof is to provide a non-invasive living body measurement device that can obtain a clear image of a finger of a subject, and whose precision in biological information detection is improved over conventional technology.

Solution to Problem

This object is accomplished by the non-invasive living body measurement device according to claim 1. The dependent claims concern particular embodiments.

A non-invasive living body measurement device includes: a platform for placement of a finger of a subject that is a measurement target; a plurality of light emitting units arranged in a line along a lengthwise direction of the finger placed on the platform; a light amount adjustment unit that adjusts a light amount of each of the plurality of light emitting units; an imaging unit that obtains an image of the finger placed on the platform by receiving transmitted light that passed through the finger when light was emitted from the light emitting units; and an information processing unit that detects biological information regarding the subject by processing the image obtained by the imaging unit.

According to this, the transmitted light amount is made uniform with respect to the lengthwise direction of the finger, thus preventing the image of the subject's finger from having locally dark and light portions, and obtaining a clear image. Also, due to the image being clear, the region of interest that is to be the image processing target is larger, thus making it possible to set a more appropriate region of interest than in conventional technology. As a result, it is possible to increase the size of the region in which an image of a blood vessel portion, which is the processing target image, can be found, and precision in biological information detection is improved over conventional technology.

In the above aspect, the light amount adjustment unit may be configured to set the light amount of a light emitting unit provided at a position closest to a joint of the finger placed on the platform lower than the light amount of another light emitting unit. Since light easily passes through the joint portion, by setting the light amount of the light emitting unit corresponding to this portion lower than the light amounts of the other light emitting units, it is possible to prevent the image of the subject's finger from having locally dark and light portions.

In the above aspect, the light amount adjustment unit may be configured to increase the light amount of each of the light emitting units with increasing distance from the joint of the finger placed on the platform. Light passes less readily with increasing distance from the joint portion of the finger, and therefore according to the above configuration, it is possible to prevent the image of the subject's finger from having locally dark and light portions.

In the above aspect, the light amount adjustment unit may be configured to adjust the light amounts of the light emitting units such that the light amount increases non-linearly with increasing distance from the joint of the finger placed on the platform. The transmittance of light changes non-linearly with increasing distance from the joint portion of the finger, and therefore according to the above configuration, the transmitted light amount is made even more uniform with respect to the lengthwise direction of the finger.

In the above aspect, the light amount adjustment unit may be configured to determine extents of change in the light amounts of the light emitting units relative to distance from the joint of the finger placed on the platform, and adjust the light amounts of the light emitting units in accordance with the determined extents of change in the light amounts.

In the above aspect, the light amount adjustment unit may be configured to adjust the light amounts of the light emitting units such that an average value of light amounts of transmitted light that passed through the finger placed on the platform is a predetermined value.

In the above aspect, the light amount adjustment unit may be configured to adjust the light amounts of the light emitting units based on a transmission image of the finger obtained by the imaging unit.

In the above aspect, the light amount adjustment unit may be configured to adjust the light amounts of the light emitting units based on luminance in the transmission image of the finger obtained by the imaging unit.

In the above aspect, the information processing unit may be configured to control the light amount adjustment unit so as to execute light amount adjustment processing on the light emitting units before image processing is performed on the finger of the subject by the imaging unit.

In the above aspect, the light emitting units are each equipped with a plurality of light emitting devices that emit light of mutually different wavelengths, and light emitting devices that emit light of the same wavelength may be arranged in a line along the lengthwise direction of the finger placed on the platform. Advantageous Effects of the Invention

According to the present invention, it is possible to obtain a clear image of a finger of a subject, and possible to improve precision in biological information detection.

Brief Description of Drawings

FIG. 1 is a perspective view of an external configuration of a non-invasive living body measurement device according to an embodiment.
FIG. 2 is a block diagram showing a configuration of the non-invasive living body measurement device according to the embodiment.
FIG. 3 is a cross-sectional side view of a configuration of a measurement unit.

FIG. 4 is a bottom view of a configuration of a light emitting unit.

FIG. 5 is a circuit diagram of the configuration of the light emitting unit.

FIG. 6 is a flowchart showing a procedure of operation of the non-invasive living body measurement device according to the embodiment.

FIG. 7 is a flowchart showing a procedure of light amount adjustment processing.

FIG. 8 is a graph for describing light emitting unit light amount adjustment.

FIG. 9 is a graph for describing light emitting unit light amount adjustment.

FIG. 10 is a diagram showing an example of a transmission image of a finger obtained by a conventional illumination method.

FIG. 11 is a diagram showing an example of a transmission image of a finger after execution of light amount adjustment processing by the non-invasive living body measurement device according to the embodiment.

FIG. 12A is a flowchart showing a procedure of hemoglobin concentration detection processing (former half).

FIG. 12B is a flowchart showing the procedure of hemoglobin concentration detection processing (latter half).

FIG. 13 is a graph showing an example of a luminance profile.

FIG. 14 is a flowchart showing a procedure of absorption profile creation and baseline determination processing

FIG. 15 is a graph showing an example of an absorption profile.

FIG. 16 is a graph showing an example of an absorption profile.

FIG. 17 is a graph showing an example of a blood vessel profile.

FIG. 18 is a flowchart showing a procedure of venous oxygenation index detection processing.

Description of Embodiments

Preferred embodiments of the present invention will be described below with reference to the diagrams.

Configuration of non-invasive living body measurement device

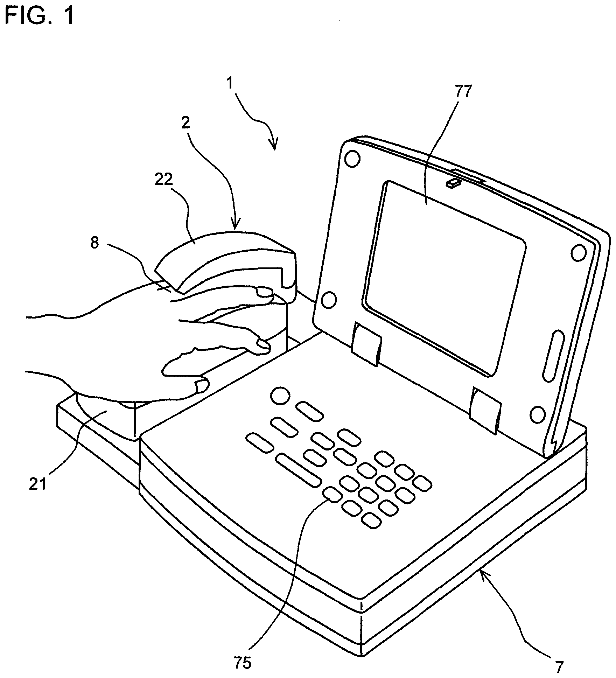

FIG. 1 is a perspective view of the external configuration of a non-invasive living body measurement device 1 according to the present embodiment. The non-invasive living body measurement device 1 includes a measurement unit 2 and an information processing unit 7. The measurement unit 2 is for capturing images of a subject's finger, and the information processing unit 7 is for detecting biological information (hemoglobin value, venous oxygenation index) regarding the subject by processing the imaging data (image data) from the measurement unit 2, and displaying the biological information as the detection result.

The measurement unit 2 includes a platform 21 on which a finger 8 of the subject can be placed, and a cover unit 22 for covering the finger 8 placed on the platform 21. The cover unit 22 is arranged above the platform 21, and is pivotally attached to the platform 21. The cover unit 22 can open and close (cover the finger 8 and expose the finger 8) by swinging relative to the platform 21.

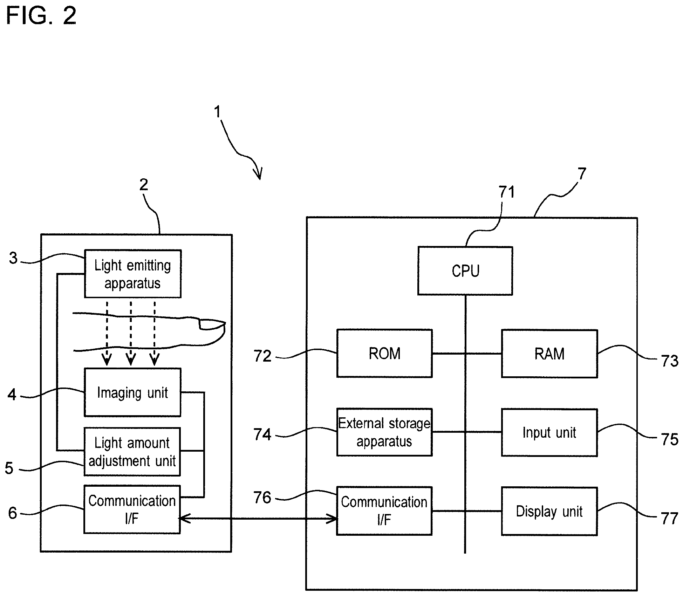

FIG. 2 is a block diagram showing the configuration of the non-invasive living body measurement device 1. The measurement unit 2 has a light emitting apparatus 3, an imaging unit 4 constituted by a CCD, a light amount adjustment unit 5 that adjusts the light emission amount of the light emitting apparatus 3, and a communication interface 6.

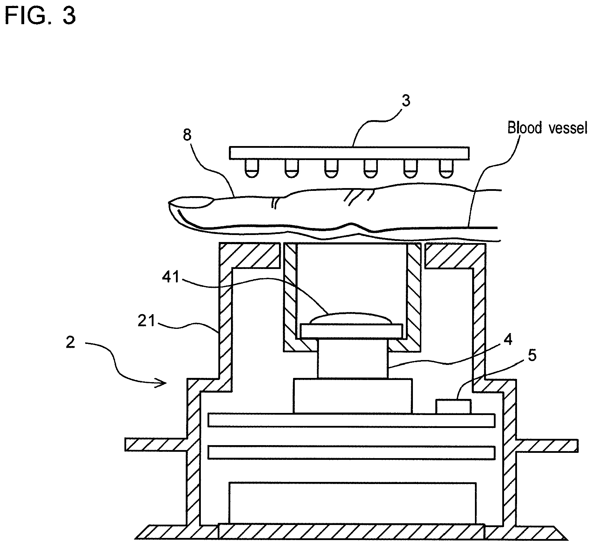

FIG. 3 is a cross-sectional side view of the configuration of the measurement unit 2. The light emitting apparatus 3 is equipped with multiple LEDs, and is arranged on the cover unit 22. A base is provided within the platform 21, and the imaging unit 4, the light amount adjustment unit 5, and the communication interface 6 are attached to the base. The imaging unit 4 is provided on the base such that the light receiving face is facing upward, and a lens system 41 is arranged above the imaging unit 4. Also, in this configuration, the light emitting apparatus 3 is located above the imaging unit 4 when the cover unit 22 is closed.

The subject's finger 8 can be placed on the platform 21 as described above. At this time, the subject's middle finger 8 is placed so as to be located directly above the imaging unit 4. Also, the platform 21 is formed so as to conform to the shape of the subject's middle finger 8, and is configured such that the second joint of the subject's middle finger 8 is located at the center of the imaging range of the imaging unit 4 when the subject's hand is placed over the platform 21.

When the subject's finger 8 is placed on the platform 21 and the cover unit 22 is closed, the light emitting apparatus 3 and the imaging unit 4 oppose each other so as to sandwich the subject's finger 8 from above and below. The light emitting apparatus 3 is configured so as to emit light downward, and the light emitted from the light emitting apparatus 3 passes through the subject's finger 8. This transmitted light passes through the lens system 41 and arrives at the imaging unit 4. In this way, a transmission image of the subject's finger 8 is obtained by the imaging unit 4.

The light amount adjustment unit 5 is constituted by an FPGA (Field-Programmable Gate Array). This light amount adjustment unit 5 receives an output signal from the imaging unit 4 and performs light amount adjustment on the light emitting apparatus 3 based on the output signal. The light amount adjustment performed on the light emitting apparatus 3 will be described in detail later.

As shown in FIG. 2, the communication interface 6 is communicably connected to the imaging unit 4 and the light amount adjustment unit 5. This communication interface 6 is constituted by a USB interface, for example, and is connected to the information processing unit 7 via a communication cable. In other words, the measurement unit 2 can exchange data with the information processing unit 7 using the communication interface 6.

The information processing unit 7 has a CPU 71, a ROM 72, a RAM 73, an external storage apparatus 74, an input unit 75, a communication interface 76, and a display unit 77.

The CPU 71 executes computer programs stored in the ROM 72 and computer programs loaded to the RAM 73. The RAM 73 is used for the reading out of computer programs stored in the ROM 72 and the external storage apparatus 74. The RAM 73 is also used as a work area for the CPU 71 when these computer programs are executed.

The external storage apparatus 74 is constituted by a non-volatile storage device such as a hard disk or a flash memory. Various computer programs to be executed by the CPU 71, such as an operating system and application programs, are installed in the external storage apparatus 74 along with data to be used in the execution of the computer programs.

The input unit 75 is an operation unit provided with multiple keys as shown in FIG. 1. By operating the keys of this input unit, a user can input various instructions, data, and the like to the non-invasive living body inspection device 1. The display unit 77 receives an image signal from the CPU 71, and displays images based on the input images signal. Also, the communication interface 76 is a USB interface, and is connected to the communication interface 6 of the measurement unit 2 via a signal cable. The information processing unit 7 can exchange data with the measurement unit 2 using the communication interface 76.

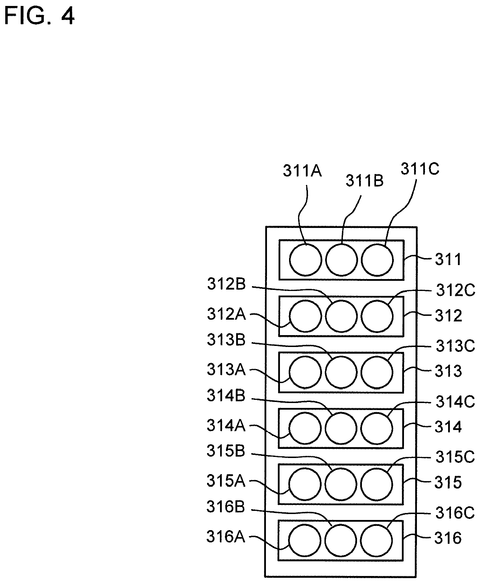

The following describes details of the configuration of the light emitting apparatus 3. FIG. 4 is a bottom view of the configuration of the light emitting apparatus 3. As shown in FIG. 4, the bottom surface of the light emitting apparatus 3 is shaped as a rectangle that is elongated in one direction. This light emitting apparatus 3 is provided with six LED blocks 311 to 316. The LED blocks 311 to 316 are arranged in a column along the lengthwise direction of the rectangular shape. This lengthwise direction matches the direction orthogonal to the shaft length direction of the rotation shaft for pivotably attaching the platform 21 and the cover unit 22, that is to say, matches the lengthwise direction of the subject's finger 8 to be placed on the platform 21. In other words, the LED blocks 311 to 316 are arranged in a column in the lengthwise direction of the rectangular shape. Note that when the cover unit 22 is closed, the center of the light emitting apparatus 3 is located at the center of the imaging range of the imaging unit 4. In other words, in this configuration, when the subject's hand is placed on the platform 21, the second joint of the subject's middle finger 8 is located at the center of the light emitting apparatus 3.

The LED blocks 311 to 316 respectively include three LEDs, namely LED 311A, LED 311B, and LED 311C to LED 316A, LED 316B, and LED 316C. The three LEDs included in the respective LED blocks 311 to 316, namely LED 311A, LED 311B, and LED 311C to LED 316A, LED 316B, and LED 316C, are arranged in a line along the short-side direction of the previously-described rectangular shape of the light emitting apparatus 3. In other words, 18 LEDs are arranged in a matrix in the light emitting apparatus 3.

Also, the LEDs 311A, 312A, 313A, 314A, 315A, and 316A are configured so as to emit light with a wavelength of 660 nm. This light with a wavelength of 660 nm is absorbed to a greater extent by reduced hemoglobin than oxygenated hemoglobin. The LEDs 311B, 312B, 313B, 314B, 315B, and 316B are configured so as to emit light with a wavelength of 805 nm. This light with a wavelength of 805 nm is absorbed to the same extent by oxygenated hemoglobin and reduced hemoglobin. Also, the LEDs 311C, 312C, 313C, 314C, 315C, and 316C are configured so as to emit light with a wavelength of 880 nm. The LEDs 311A, 312A, 313A, 314A, 315A, and 316A that emit light with a wavelength of 660 nm and the LEDs 311B, 312B, 313B, 314B, 315B, and 316B that emit light with a wavelength of 805 nm are used to measure a venous oxygenation index. The LEDs 311B, 312B, 313B, 314B, 315B, and 316B that emit light with a wavelength of 805 nm and the LEDs 311C, 312C, 313C, 314C, 315C, and 316C that emit light with a wavelength of 880 nm are used to measure a hemoglobin concentration.

Also, the LEDs 311A, 312A, 313A, 314A, 315A, and 316A that emit light with a wavelength of 660 nm are arranged in a column along the lengthwise direction of the light emitting apparatus 3. Similarly, the LEDs 311B, 312B, 313B, 314B, 315B, and 316B that emit light with a wavelength of 805 nm are also arranged in a column along the lengthwise direction of the light emitting apparatus 3, and the LEDs 311C, 312C, 313C, 314C, 315C, and 316C that emit light with a wavelength of 880 nm are also arranged in a column along the lengthwise direction of the light emitting apparatus 3.

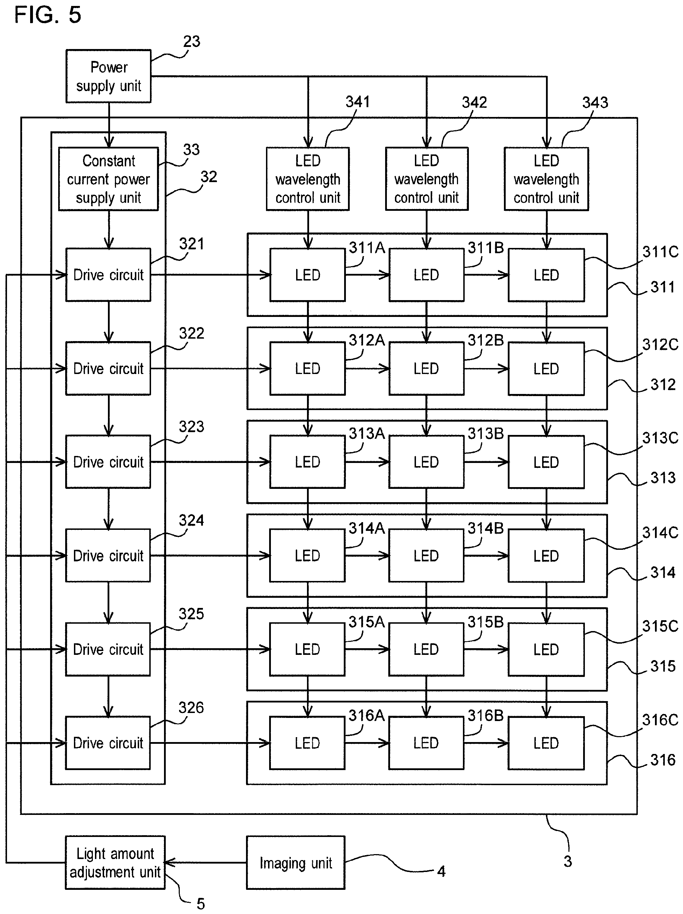

FIG. 5 is a circuit diagram of the configuration of the light emitting apparatus 3. As shown in FIG. 5, the light emitting apparatus 3 is equipped with a drive unit 32 for driving the LEDs 311A to 316A, 311B to 316B, and 311C to 316C. The drive unit 32 is provided with drive circuits 321 to 326 for respectively driving the LED blocks 311 to 316. In other words, the LED blocks 311 to 316 are individually driven by the drive circuits 321 to 326. Also, the drive unit 32 is provided with a constant current power supply unit 33. A power supply unit 23 provided in the platform 21 is connected to the constant current power supply unit 33, and constant current is supplied from the constant current power supply unit 33 to the drive circuits 321 to 326. Also, the power supply unit 23 is connected to each of three LED wavelength control units 341, 342, and 343 provided in the light emitting apparatus 3. The LED wavelength control unit 341 is connected to the LEDs 311A, 312A, 313A, 314A, 315A, and 316A that emit light with a wavelength of 660 nm, and performs control such that the emitted light wavelength of these LEDs 311A, 312A, 313A, 314A, 315A, and 316A is 660 nm. Similarly, the LED wavelength control unit 342 is connected to the LEDs 311B, 312B, 313B, 314B, 315B, and 316B that emit light with a wavelength of 805 nm, and performs control such that the emitted light wavelength of these LEDs 311B, 312B, 313B, 314B, 315B, and 316B is 805 nm. Also, the LED wavelength control unit 343 is connected to the LEDs 311C, 312C, 313C, 314C, 315C, and 316C that emit light with a wavelength of 880 nm, and performs control such that the emitted light wavelength of these LEDs 311C, 312C, 313C, 314C, 315C, and 316C is 880 nm.

The light amount adjustment unit 5 is connected to each of the drive circuits 321 to 326. This light amount adjustment unit 5 outputs a PWM signal to each of the drive circuits 321 to 326. The drive circuits 321 to 326 each supply a current that corresponds to the duty ratio of the received PWM (Pulse Width Modulation) signal to the corresponding one of the LED blocks 311 to 316. The light emission amounts of the LED blocks 311 to 316 are thus adjusted individually. In this case, current with the same value is supplied to each of the three LEDs provided in the same LED block. In other words, the three LEDs in the same LED block are subjected to light amount adjustment in an integrated manner.

Operation of non-invasive living body measurement device

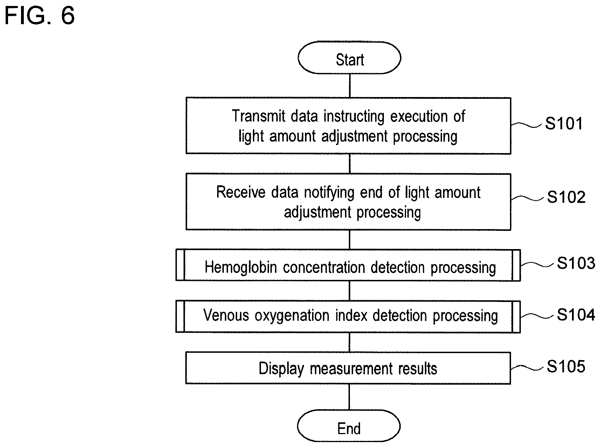

Next, operation of the non-invasive living body measurement device 1 according to the present embodiment will be described. FIG. 6 is a flowchart showing a procedure of operation of the non-invasive living body measurement device 1 according to the present embodiment. When a subject is to use the non-invasive living body measurement device 1, firstly, the subject's middle finger 8 is placed on the platform 21. At this time, the second joint of the subject's middle finger 8 is located at the central position in the imaging range of the imaging unit 4. In this state, the subject inputs a measurement start instruction to the non-invasive living body measurement device 1 by operating the input unit 75 of the information processing unit 7. When the measurement start instruction is given to the non-invasive living body measurement device 1, the CPU 71 of the information processing unit 7 transmits data for instructing execution of light amount adjustment processing on the light emitting apparatus 3 to the measurement unit 2 (step S101).

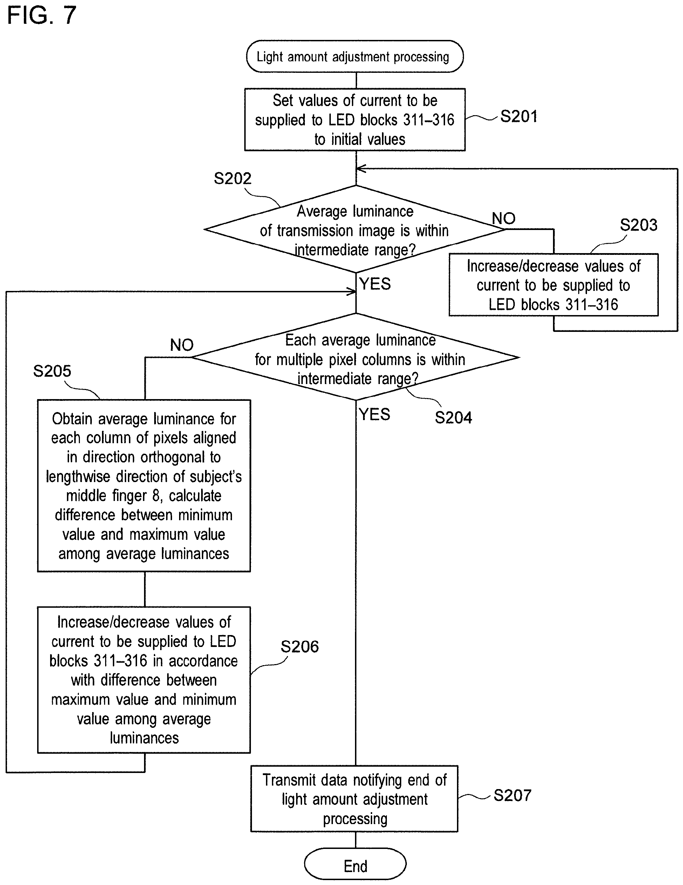

The light amount adjustment unit 5 of the measurement unit 2 receives the execution instruction data and executes light amount adjustment processing. FIG. 7 is a flowchart showing a procedure of light amount adjustment processing. First, the light amount adjustment unit 5 sets initial values for the values of current to be supplied to (light amounts of) the LED blocks 311 to 316 (step S201). According to these initial values, the values of current to be supplied to the LED blocks 311 to 316 are set such that among the LED blocks 311 to 316, the light amount is the smallest for the LED blocks 313 and 314 that are the closest to the second joint of the subject's middle finger 8, and the light amount increases with increasing distance from the LED blocks 313 and 314. A transmission image of the subject's middle finger 8 is obtained by the imaging unit 4 in this state, and the transmission image is given to the light amount adjustment unit 5. The transmission image contains a transmission image whose range is from the vicinity of the first joint of the subject's middle finger 8 to the third joint (base). The transmission image is a 256-level monochrome image. The light amount adjustment unit 5 obtains an average luminance for all of the pixels in the transmission image, and determines whether the average luminance falls within a predetermined intermediate range centered on 127, which is the median value of the 256 levels (step S202).

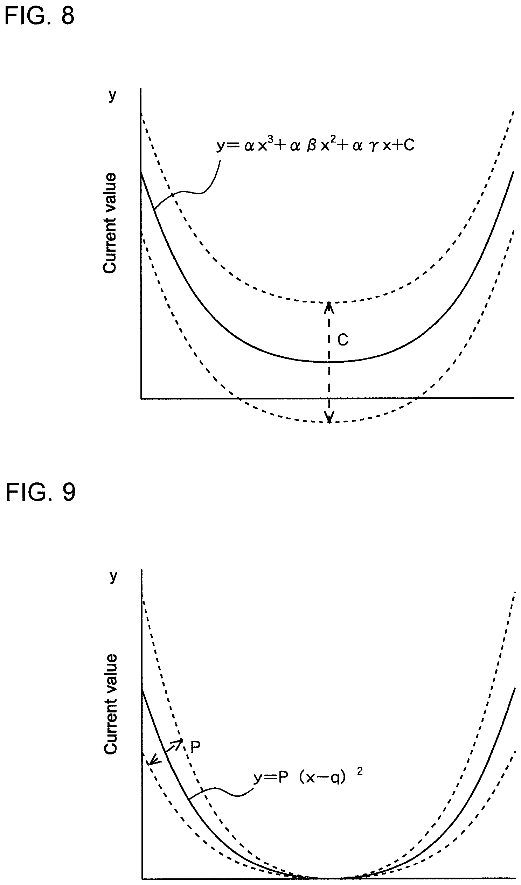

If the average luminance is outside the intermediate range (NO in step S202), the light amount adjustment unit 5 increases or decreases the values of current to be supplied to (light amounts of) the LED blocks 311 to 316 (step S203). Specifically, if the average luminance is lower than the intermediate range, the light amounts of the LED blocks 311 to 316 are increased, and if the average luminance is higher than the intermediate range, the light amounts of the LED blocks 311 to 316 are decreased. FIG. 8 is a graph for describing light amount adjustment performed on the light emitting apparatus 3 in step S203. In FIG. 8, the vertical axis y indicates the current value, and the horizontal axis x indicates the LED block position. As shown in FIG. 8, in the processing of step S203, the values of current to be supplied to the LED blocks 311 to 316 are given by the following cubic equation 1.

$$y = \alpha x3 + \alpha\beta x2 + \alpha\gamma x + C \ldots (1)$$

In the processing of step S203, the values of current to be supplied to the LED blocks 311 to 316 are adjusted by changing the parameter C in the equation 1 above. In other words, the overall light amount of the LED blocks 311 to 316 is increased or decreased while maintaining the light amount differences between the LED blocks 311 to 316.

After the processing of step S203 is performed, the light amount adjustment unit 5 returns to the processing of step S202. Accordingly, the processing of steps S202 and S203 is repeated until the average luminance falls within the intermediate range.

On the other hand, if the average luminance is within the intermediate range in step S202 (YES in step S202), the light amount adjustment unit 5 obtains an average luminance for each of a predetermined number of (two or more) columns of pixels aligned in the direction orthogonal to the lengthwise direction of the subject's middle finger 8 in the transmission image, and determines whether each of the average luminances falls within the intermediate range (step S204).

If any of the average luminances is outside the intermediate range in step S204 (NO in step S204), the light amount adjustment unit 5 obtains an average luminance for each of the columns of pixels aligned in the direction orthogonal to the lengthwise direction of the subject's middle finger 8, and calculates the difference between the maximum value and the minimum value among the average luminances (step S205). Next, the light amount adjustment unit 5 increases or decreases the values of current to be supplied to the LED blocks 311 to 316 in accordance with the calculated difference between the maximum value and minimum value among the average luminances (step S206). FIG. 9 is a graph for describing light amount adjustment performed on the light emitting apparatus 3 in step S206. In FIG. 9, the vertical axis y indicates the current value, and the horizontal axis x indicates the LED block position. As shown in FIG. 9, in the processing of step S206, the values of current to be supplied to the LED blocks 311 to 316 are given by the following quadratic equation 2.

$$y = P(x-q)2 \ ... \ (2)$$

In the processing of step S206, the values of current to be supplied to the LED blocks 311 to 316 are adjusted by changing the parameter P in the equation 2 above. Specifically, as shown in FIG. 9, if the difference between the maximum value and minimum value among the average luminances is larger than a predetermined value, the parameter P is adjusted such that the curvature of the parabola decreases, and if the difference between the maximum value and minimum value among the average luminances is smaller than the predetermined value, the parameter P is adjusted such that the curvature of the parabola increases.

After the processing of step S206 is performed, the light amount adjustment unit 5 returns to the processing of step S204. Accordingly, the processing of steps S204 to S206 is repeated until the average luminances fall within the intermediate range.

If all of the average luminances are within the intermediate range in step S204 (YES in step S204), the light amount adjustment unit 5 transmits data notifying the end of light amount adjustment processing to the information processing unit 7 (step S207), and ends the light amount adjustment processing.

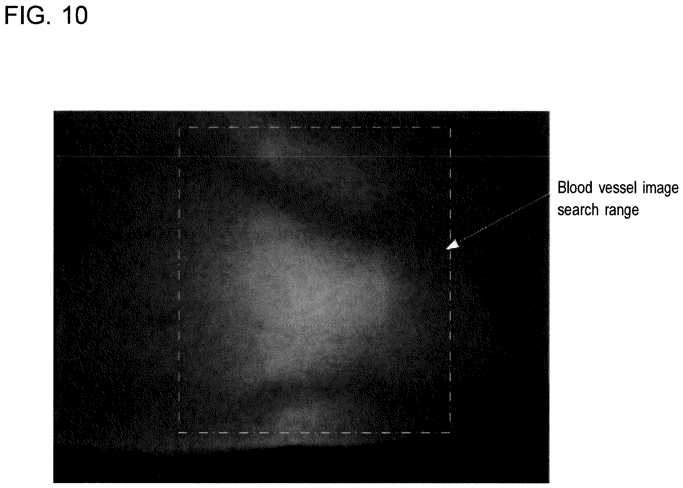

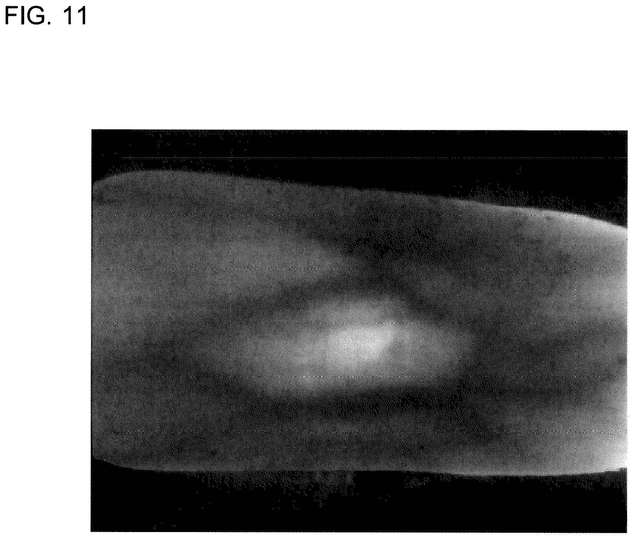

FIG. 10 is a diagram showing an example of a transmission image of a finger obtained by a conventional illumination method, and FIG. 11 is a diagram showing an example of a transmission image of a finger after execution of light amount adjustment processing by the non-invasive living body measurement device 1 according to the present embodiment. It can be seen that light more easily passes through the joint portion of the finger than the other portions. As shown in FIG. 10, with a conventional illumination method, the light amounts of all of the light emitting devices are set the same, and therefore the joint portion of the finger is much brighter than the other portions. As will be described later, in the detection of the hemoglobin concentration, it is necessary to find an image of a blood vessel portion (hereinafter, referred to as "blood vessel image") in the transmission image and set the blood vessel image as the processing target. With the transmission image obtained by a conventional illumination method, it is necessary to find a blood vessel image in the portion of the transmission image corresponding to the joint portion of the finger. In other words, the region in which the blood vessel image can be found is limited to the joint portion of the finger, and there is a risk of not being able to find an appropriate blood vessel image. In contrast, with the transmission image of the finger after execution of light amount adjustment processing in the present embodiment, the obtained image is an overall clear image that does not have locally dark and light portions. For this reason, the search range for the blood vessel image is larger than in conventional technology, and an appropriate blood vessel image is easier to find.

As shown in FIG. 6, if the information processing unit 7 receives light amount adjustment processing end notification data (step S102), the CPU 71 executes hemoglobin concentration detection processing as will be described below (step S103).

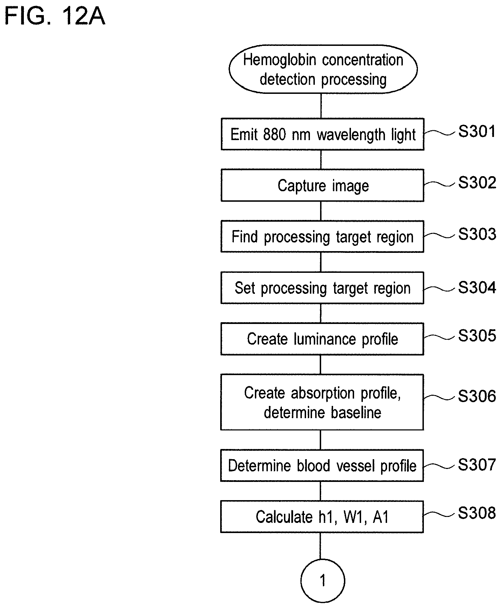

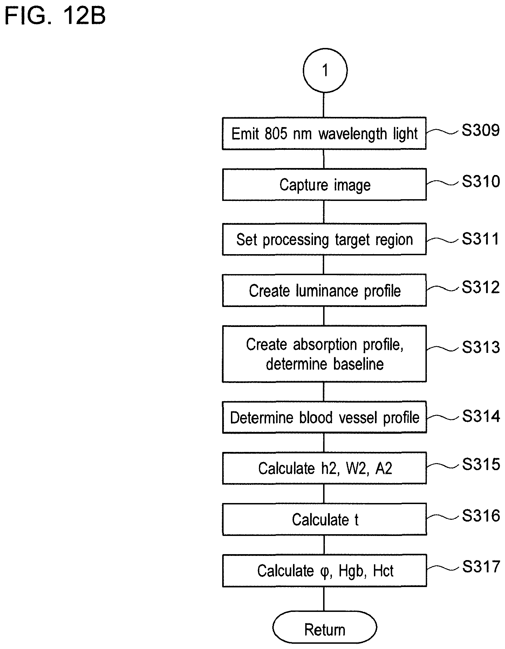

FIGS. 12A and 12B are flowchart showing a procedure of hemoglobin concentration detection processing. In hemoglobin concentration detection processing, firstly, the CPU 71 causes the LEDs 311C, 312C, 313C, 314C, 315C, and 316C to irradiate the subject's finger 8 with light with a wavelength of 880 nm (step S301), and causes the imaging unit 4 to capture an image of the finger 8 (step S302). The CPU 71 then searches for the blood vessel image region having the highest contrast in the transmission image for 880 nm wavelength light captured by the imaging unit 4 (hereinafter, referred to as "first transmission image") (step S303), and sets the found region as the processing target region (step S304).

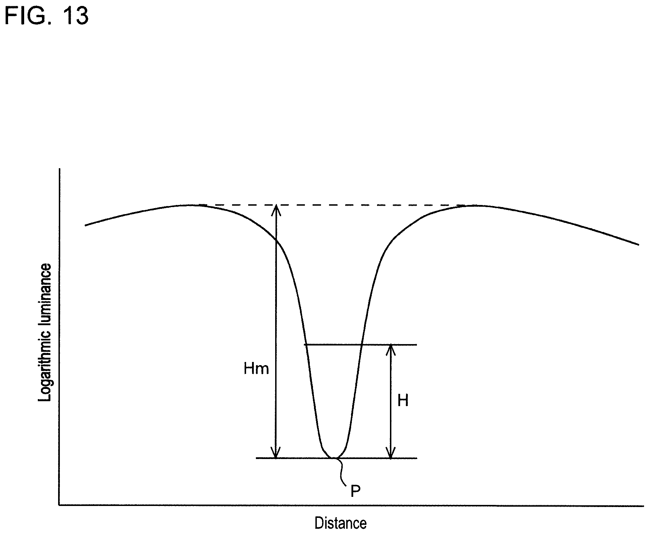

Next, the CPU 71 performs logarithmic transformation on a luminance distribution whose range crosses a blood vessel in the processing target region, and, based on the transformed image luminance distribution, creates a valley-shaped luminance profile whose local minimum is at the center of the blood vessel (step S305). FIG. 13 is a graph showing an example of a luminance profile. In FIG. 13, the vertical axis indicates logarithmic luminance, and the horizontal axis indicates distance. Subsequently, the CPU 71 performs absorption profile creation and baseline determination based on the obtained luminance profile (step S306).

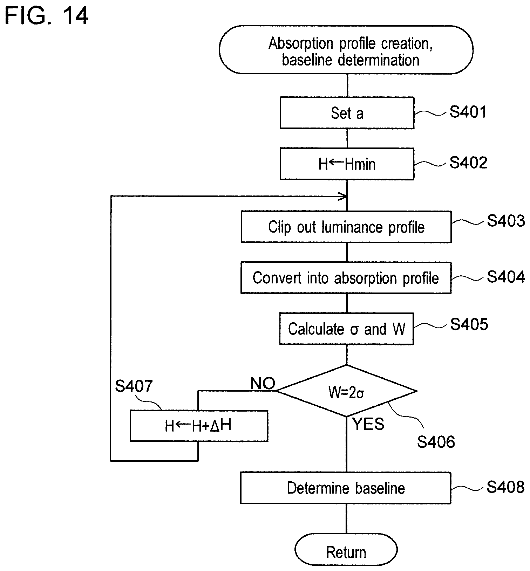

The following describes details of the processing of step S306. FIG. 14 is a flowchart showing a procedure of absorption profile creation and baseline determination processing. First, the CPU 71 sets a constant a (e.g., 0.7) (step S401).

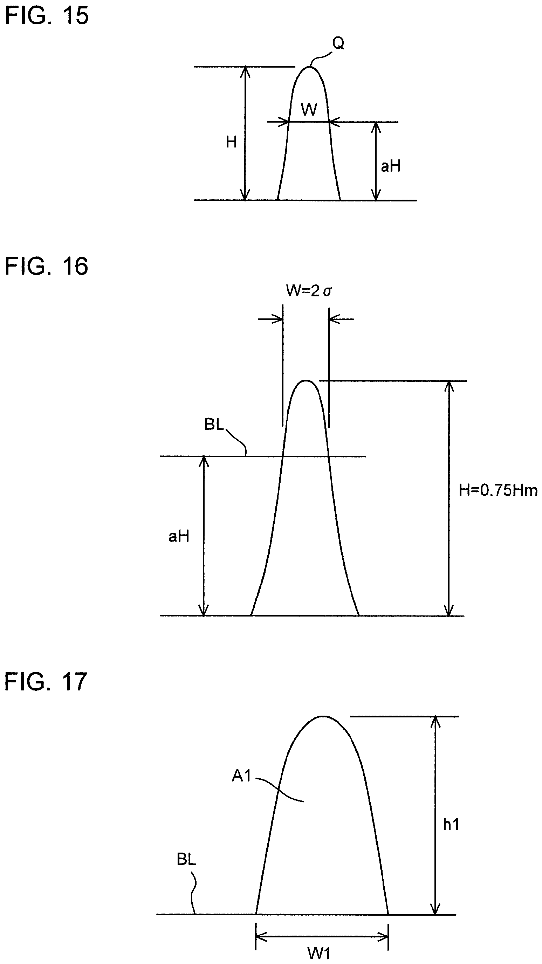

Next, the CPU 71 sets H to a predetermined minimum value (e.g., 10% of a maximum depth Hm in the luminance profile) (step S402). The CPU 71 then clips out the portion from a valley bottom P to the depth H from the luminance profile (step S403), and converts the clipped-out portion into an absorption profile (step S404). FIG. 15 is a graph showing an example of an absorption profile.

Next, the CPU 71 considers the absorption profile to be a distribution function (probability density function), and calculates a standard deviation $\sigma$ thereof and a width W at a height aH in the absorption profile (step S405).

Next, the CPU 71 determines whether $2\sigma$ and W match (step S406), and if they do not match (NO in step S406), re-sets H to a slightly higher value (step S407), and then returns to the processing of step S403.

As steps S403 to S406 are repeated, $2\sigma$ and W each change along with the increase in H, and when H reaches 75% of Hm, $2\sigma$ and W match each other, that is to say $2\sigma$=W. The absorption profile obtained at this time is shown in FIG. 16, and the CPU 71 determines the horizontal line at the height 0.7H as a baseline BL (step S408), and then returns to the call address of the absorption profile creation and baseline determination processing in hemoglobin concentration detection processing.

Next, the CPU 71 clips out the portion above the baseline BL in the absorption profile in FIG. 16, as shown in FIG. 17, and determines the clipped-out portion as the blood vessel profile (step S307). A height h1, a width W1, and an area A1 of the blood vessel profile shown in FIG. 17 are then calculated (step S308).

Next, the CPU 71 causes the LEDs 311B, 312B, 313B, 314B, 315B, and 316B to irradiate the subject's finger 8 with light with a wavelength of 805 nm (step S309), and causes the imaging unit 4 to capture an image of the finger 8 (step S310). The CPU 71 then searches for the same region as in step S304 in the transmission image for 805 nm wavelength light (hereinafter, referred to as "second transmission image") captured by the imaging unit 4, and sets the found region as the processing target region (step S311).

Thereafter, in steps S312 to S315, processing similar to the processing performed on the first transmission image in steps S305 to S308 is performed on the second transmission image, and a height h2, a width W2, and an area A2 are calculated for the blood vessel profile corresponding to the wavelength of 805 nm.

The CPU 71 then calculates a depth t from the tissue surface to the blood vessel using the following equation (step S316).

$$t=(h2/W2n)/(h1/W1)m \ \dots \ (3)$$

Note that m and n are constants.

Next, the CPU 71 calculates a blood vessel diameter $\varphi$ as well as a hemoglobin concentration Hgb and a hematocrit value Hct of the blood flowing through the blood vessel using equations 4 to 6 respectively (step S317).

$$\varphi=A1p/h1=W1\times f1(t) \ \dots \ (4)$$

$$Hgb=(h1/A1q)\times f2(t)\times g(\varphi) \ \dots \ (5)$$

Note that p and q are constants, and the functions f1, f2, and g are functions determined experimentally.

$$Hct=k\cdot h1/h2+L \ \dots \ (6)$$

Note that k and L are constants.

After the blood vessel depth, blood vessel diameter, hemoglobin concentration, and hematocrit value are obtained as described above, the CPU 71 returns to the call address of hemoglobin concentration detection processing in the main routine.

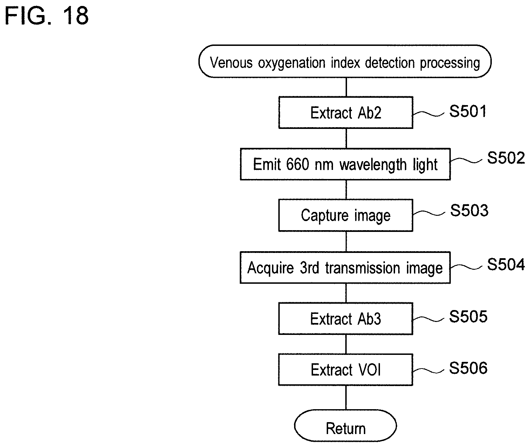

When the hemoglobin concentration detection processing ends, as shown in FIG. 6, the CPU 71 executes venous oxygenation index detection processing (step S104). FIG. 18 is a flowchart showing a procedure of venous oxygenation index detection processing. In venous oxygenation index detection processing, firstly, the CPU 71 extracts an absorbance Ab2 for 805 nm wavelength light from the second transmission image acquired in hemoglobin concentration detection processing (step S501).

Next, the CPU 71 causes the LEDs 311A, 312A, 313A, 314A, 315A, and 316A to irradiate the subject's finger 8 with light with a wavelength of 660 nm (step S502), and causes the imaging unit 4 to capture an image of the finger 8 (step S503). The CPU 71 then acquires a transmission image for 660 nm wavelength light (hereinafter, referred to as "third transmission image") captured by the imaging unit 4 (step S504), and extracts an absorbance Ab3 for 660 nm wavelength light from the acquired third transmission image (step S505).

The CPU 71 then calculates a venous oxygenation index VOI using the following equation 7 (step S506).

$$VOI = -Ab3/Ab2 \ ... \ (7)$$

After the venous oxygenation index is obtained as described above, the CPU 71 returns to the call address of venous oxygenation index detection processing in the main routine.

When venous oxygenation index detection processing ends, as shown in FIG. 6, the hemoglobin concentration, hematocrit value, and venous oxygenation index obtained by the CPU 71 are displayed on the display unit 77 (step S105), and then this processing ends.

Other Embodiments

Note that although the above embodiment describes a configuration in which the light amounts of the LED blocks 311 to 316 provided in the light emitting apparatus 3 are independently controlled by the light amount adjustment unit 5, there is no limitation to this. A configuration is possible in which the light amounts of all of the LED blocks 311 to 316 are increased or decreased in an integrated manner. In this case, the LED blocks 311 to 316 are set such that the light amount increases with increasing distance from the LED blocks 313 and 314 that are the closest to the joint of the subject's finger, and the overall light amount of the LED blocks 311 to 316 is increased or decreased while maintaining the light amount differences between the LED blocks 311 to 316.

Also, although the above embodiment describes a configuration in which the light amounts of the LED blocks 311 to 316 are adjusted such that the light amount increases non-linearly with increasing distance from the joint of the finger placed on the platform 21, there is no limitation to this. A configuration is possible in which the light amounts of the LED blocks 311 to 316 are adjusted such that the light amount increases linearly with increasing distance from the joint of the finger placed on the platform 21.

Also, although the above embodiment describes a configuration in which the light amounts of the LED blocks 311 to 316 are adjusted such that the light amount increases with increasing distance from the joint of the finger placed on the platform 21, there is no limitation to this. For example, if a joint is not present at the measurement target site, there is no limitation to a configuration in which the light amounts of the LED blocks 311, 312, 315, and 316 are determined such that the light amounts of the central LED blocks 313 and 314 are low, and the light amount increases with increasing distance from the center, and it is sufficient that the light amounts of the LED blocks are adjusted such that the transmitted light amount is uniform in the imaging range.

Also, although the above embodiment describes a configuration in which the light amount adjustment unit 5 that performs light amount adjustment and the CPU 71 of the information processing unit 7 that performs image processing are provided separately, there is no limitation to this. A configuration is possible in which light amount adjustment processing and image processing are executed by one arithmetic processing unit.

Also, although the above embodiment describes a configuration provided with the LED blocks 311 to 316 arranged in a column, there is no limitation to this, and a configuration is possible in which the LED blocks are arranged in a zigzag line, for example.

Also, although the above embodiment describes a configuration in which a finger of the subject is the imaging target, there is no limitation to this. A configuration is possible in which a toe of the subject is imaged, and the hemoglobin concentration of the subject is detected based on the thus-obtained image.

Industrial Applicability

A non-invasive living body measurement device according to the present invention is useful as a non-invasive living body measurement device that irradiates a finger of a subject with light and obtains transmitted light, captures an image of the finger by receiving the transmitted light with an imaging unit, and detects biological information regarding the subject by processing the obtained image.

List of Reference Numerals

| | |
|---|---|
| 1 | Non-invasive living body measurement device |
| 2 | Measurement unit |
| 21 | Platform |
| 3 | Light emitting apparatus |
| 311-316 | LED block |
| 311A-316A, 311B-316B, 311C-316C | LED |
| 4 | Imaging unit |
| 5 | Light amount adjustment unit |
| 7 | Information processing unit |
| 71 | CPU |

**Claims**

1. A non-invasive living body measurement device (1) comprising:

   a platform (21) for placement of a finger (8) of a subject that is a measurement target;
   a plurality of light emitting units (311-316) arranged in a line along a lengthwise direction of the finger placed on the platform;
   a light amount adjustment unit (5) configured to adjust a light amount of each of the plurality of light emitting units;
   an imaging unit (4) configured to obtain an image of the finger placed on the platform by receiving transmitted light that passed through the finger when light is emitted from the light emitting units; and
   an information processing unit (7) configured to detect biological information regarding the subject by processing the image obtained by the imaging unit, wherein
   the biological information is a hemoglobin value or a venous oxygenation index, and
   the light emitting units (311-316) are each equipped with a plurality of light emitting devices (311A, 311B, 311C; ... ;316A, 316B, 316C) configured to emit light of mutually different wavelengths.

2. The non-invasive living body measurement device (1) according to claim 1, wherein the light amount adjustment unit (5) is configured to increase the light amount of each of the light emitting units with increasing distance from the joint of the finger placed on the platform.

3. The non-invasive living body measurement device (1) according to claim 2, wherein the light amount adjustment unit (5) is configured to adjust the light amounts of the light emitting units such that the light amount increases non-linearly with increasing distance from the joint of the finger placed on the platform.

4. The non-invasive living body measurement device (1) according to claim 2 or 3, wherein the light amount adjustment unit (5) is configured to determine extents of change in the light amounts of the light emitting units relative to distance from the joint of the finger placed on the platform, and adjust the light amounts of the light emitting units in accordance with the determined extents of change in the light amounts.

5. The non-invasive living body measurement device (1) according to any one of claims 1 to 4, wherein the light amount adjustment unit (5) is configured to adjust the light amounts of the light emitting units such that an average value of light amounts of transmitted light that passed through the finger placed on the platform is a predetermined value.

6. The non-invasive living body measurement device (1) according to any one of claims 1 to 5, wherein the light amount adjustment unit (5) is configured to adjust the light amounts of the light emitting units based on a transmission image of the finger obtained by the imaging unit.

7. The non-invasive living body measurement device (1) according to claim 6, wherein the light amount adjustment unit (5) is configured to adjust the light amounts of the light emitting units based on luminance in the transmission image of the finger obtained by the imaging unit.

8. The non-invasive living body measurement device (1) according to any one of claims 1 to 7, wherein the information processing unit (5) is configured to control the light amount adjustment unit so as to execute light amount adjustment processing on the light emitting units before image processing is performed on the finger of the subject by the imaging unit.

**Patentansprüche**

1. Nichtinvasive Messvorrichtung für Lebendkörper (1), umfassend:

   eine Plattform (21) zum Platzieren eines Fingers (8) eines Subjekts, das ein Messzielobjekt ist;
   eine Vielzahl von lichtemittierenden Einheiten (311-316), die in einer Linie entlang einer Längsrichtung des auf der Plattform platzierten Fingers eingerichtet sind;
   eine Lichtmengeneinstelleinheit (5), die konfiguriert ist, um eine Lichtmenge jeder der Vielzahl von lichtemittierenden Einheiten einzustellen;
   eine Bildgebungseinheit (4), die konfiguriert ist, um ein Bild des auf der Plattform platzierten Fingers durch das Empfangen von übertragenem Licht, das den Finger passiert hat, wenn Licht von den lichtemittierenden Einheiten emittiert wird, zu erhalten; und
   eine Informationsverarbeitungseinheit (7), die konfiguriert ist, um eine biologische Information in Bezug auf das Subjekt durch Verarbeiten des von der Bildgebungseinheit erhaltenen Bildes zu detektieren, wobei
   die biologische Information ein Hämoglobinwert oder ein venöser Sauerstoffanreicherungsindex ist, und
   die lichtemittierenden Einheiten (311-316) jeweils mit einer Vielzahl von lichtemittierenden Vorrichtungen (311A, 311B, 311C; ... ; 316A, 316B, 316C) ausgestattet sind, die konfiguriert sind, um Licht mit voneinander unterschiedlichen Wellenlängen zu emittieren.

2. Nichtinvasive Messvorrichtung für Lebendkörper (1) nach Anspruch 1, wobei die Lichtmengeneinstelleinheit (5) konfiguriert ist, um die Lichtmenge jeder der lichtemittierenden Einheiten mit zunehmendem Abstand vom Gelenk des auf der Plattform platzierten Fingers zu erhöhen.

3. Nichtinvasive Messvorrichtung für Lebendkörper (1) nach Anspruch 2, wobei die Lichtmengeneinstelleinheit (5) konfiguriert ist, um die Lichtmengen der lichtemittierenden Einheiten einzustellen, so dass die Lichtmenge nicht linear mit dem zunehmenden Abstand vom Gelenk des auf der Plattform platzierten Fingers zunimmt.

4. Nichtinvasive Messvorrichtung für Lebendkörper (1) nach Anspruch 2 oder 3, wobei die Lichtmengeneinstelleinheit (5) konfiguriert ist, um Ausmaße einer Veränderung der Lichtmengen der lichtemittierenden Einheiten relativ zum Abstand vom Gelenk des auf der Plattform platzierten Fingers zu bestimmen und die Lichtmengen der lichtemittierenden Einheiten in Übereinstimmung mit den bestimmten Ausmaßen einer Veränderung der Lichtmengen einzustellen.

5. Nichtinvasive Messvorrichtung für Lebendkörper (1) nach einem der Ansprüche 1 bis 4, wobei die Lichtmengeneinstelleinheit (5) konfiguriert ist, um die Lichtmengen der lichtemittierenden Einheiten einzustellen, so dass ein Durchschnittswert von Lichtmengen von übertragenem Licht, das den auf der Plattform platzierten Finger passiert hat, ein vorgegebener Wert ist.

6. Nichtinvasive Messvorrichtung für Lebendkörper (1) nach einem der Ansprüche 1 bis 5, wobei die Lichtmengeneinstelleinheit (5) konfiguriert ist, um die Lichtmengen der lichtemittierenden Einheiten basierend auf einem Übertragungsbild des Fingers, das durch die Bildgebungseinheit erhalten wird, einzustellen.

7. Nichtinvasive Messvorrichtung für Lebendkörper (1) nach Anspruch 6, wobei die Lichtmengeneinstelleinheit (5) konfiguriert ist, um die Lichtmengen der lichtemittierenden Einheiten basierend auf einer Leuchtdichte in dem Übertragungsbild des Fingers, das durch die Bildgebungseinheit erhalten wird, einzustellen.

8. Nichtinvasive Messvorrichtung für Lebendkörper (1) nach einem der Ansprüche 1 bis 7, wobei die Informationsverarbeitungseinheit (5) konfiguriert ist, um die Lichtmengeneinstelleinheit zu steuern, so dass eine Lichtmengeneinstellverarbeitung an den lichtemittierenden Einheiten ausgeführt wird, bevor eine Bildverarbeitung an dem Finger des Subjekts durch die Bildgebungseinheit durchgeführt wird.

**Revendications**

1. Dispositif non invasif de mesure d'organisme vivant (1) comprenant :

   une plate-forme (21) pour placer un doigt (8) d'un sujet qui est une cible de mesure ;
   une pluralité d'unités d'émission de lumière (311-316) disposées en ligne le long d'une direction longitudinale du doigt placé sur la plate-forme ;
   une unité de réglage de quantité de lumière (5) configurée pour ajuster une quantité de lumière de chacune de la pluralité d'unités d'émission de lumière ;
   une unité d'imagerie (4) configurée pour obtenir une image du doigt placé sur la plate-forme en recevant une lumière transmise qui est passée à travers le doigt lorsque de la lumière est émise par les unités d'émission de lumière ; et
   une unité de traitement d'informations (7) configurée pour détecter des informations biologiques concernant le sujet en traitant l'image obtenue par l'unité d'imagerie, dans lequel
   l'information biologique est une valeur d'hémoglobine ou un indice d'oxygénation veineuse, et
   les unités d'émission de lumière (311-316) sont chacune équipées avec une pluralité de dispositifs émetteurs de lumière (311A, 311B, 311C ; ... ; 316A, 316B, 316C) configurés pour émettre une lumière de longueurs d'onde mutuellement différentes.

2. Dispositif non invasif de mesure d'organisme vivant (1) selon la revendication 1, dans lequel l'unité de réglage de quantité de lumière (5) est configurée pour augmenter la quantité de lumière de chacune des unités d'émission de lumière avec une distance croissante à partir de l'articulation du doigt placé sur la plate-forme.

3. Dispositif non invasif de mesure d'organisme vivant (1) selon la revendication 2, dans lequel l'unité de réglage de quantité de lumière (5) est configurée pour ajuster les quantités de lumière des unités d'émission de lumière de telle sorte que la quantité de lumière augmente de manière non linéaire avec une distance croissante à partir de l'articulation du doigt placé sur la plateforme.

4. Dispositif non invasif de mesure d'organisme vivant (1) selon la revendication 2 ou 3, dans lequel l'unité de réglage de quantité de lumière (5) est configurée pour déterminer des ampleurs de variation de la quantité de lumière des unités d'émission de lumière par rapport à la distance à partir de l'articulation du doigt placé sur la plate-forme, et ajuster les quantités de lumière des unités d'émission de lumière en fonction des ampleurs de variation déterminées des quantités de lumière.

5. Dispositif non invasif de mesure d'organisme vivant (1) selon l'une quelconque des revendications 1 à 4, dans lequel l'unité de réglage de quantité de lumière (5) est configurée pour ajuster les quantités de lumière des unités d'émission de lumière de sorte qu'une valeur moyenne de quantités de lumière de lumière transmise qui passe au travers du doigt placé sur la plate-forme est une valeur prédéterminée.

6. Dispositif non invasif de mesure d'organisme vivant (1) selon l'une quelconque des revendications 1 à 5, dans lequel l'unité de réglage de quantité de lumière (5) est configurée pour ajuster les quantités de lumière des unités d'émission de lumière sur la base d'une image de transmission du doigt obtenue par l'unité d'imagerie.

7. Dispositif non invasif de mesure d'organisme vivant (1) selon la revendication 6, dans lequel l'unité de réglage de quantité de lumière (5) est configurée pour ajuster les quantités de lumière des unités d'émission de lumière sur la base de la luminance de l'image de transmission du doigt obtenue par l'unité d'imagerie.

8. Dispositif non invasif de mesure d'organisme vivant (1) selon l'une quelconque des revendications 1 à 7, dans lequel l'unité de traitement d'informations (5) est configurée pour commander l'unité d'ajustement de lumière pour exécuter un traitement d'ajustement de lumière sur les unités d'émission de lumière avant le traitement d'image sur le doigt du sujet par l'unité d'imagerie.

FIG. 1

FIG. 2

FIG. 3

FIG. 4

FIG. 5

FIG. 6

```
                    ┌──────────┐
                    │  Start   │
                    └──────────┘
                         │
  ┌──────────────────────────────────────────┐
  │  Transmit data instructing execution of   │ ~ S101
  │  light amount adjustment processing        │
  └──────────────────────────────────────────┘
                         │
  ┌──────────────────────────────────────────┐
  │  Receive data notifying end of light amount│ ~ S102
  │  adjustment processing                     │
  └──────────────────────────────────────────┘
                         │
  ║┌─────────────────────────────────────────┐║
  ║│ Hemoglobin concentration detection processing│║ ~ S103
  ║└─────────────────────────────────────────┘║
                         │
  ║┌─────────────────────────────────────────┐║
  ║│ Venous oxygenation index detection processing│║ ~ S104
  ║└─────────────────────────────────────────┘║
                         │
  ┌──────────────────────────────────────────┐
  │       Display measurement results          │ ~ S105
  └──────────────────────────────────────────┘
                         │
                    ┌──────────┐
                    │   End    │
                    └──────────┘
```

## FIG. 7

Light amount adjustment processing

Set values of current to be
supplied to LED blocks 311–316
to initial values — S201

S202

Average luminance
of transmission image is within
intermediate range? — NO

S203

Increase/decrease values of
current to be supplied to
LED blocks 311–316

YES

NO

Each average luminance
for multiple pixel columns is within
intermediate range? — S204

YES

S205

Obtain average luminance for
each column of pixels aligned
in direction orthogonal to
lengthwise direction of subject's
middle finger 8, calculate
difference between minimum
value and maximum value
among average luminances

Increase/decrease values of
current to be supplied to LED
blocks 311–316 in accordance
with difference between
maximum value and minimum
value among average
luminances — S206

Transmit data notifying end of
light amount adjustment
processing — S207

End

## FIG. 8

$$y = \alpha x^3 + \alpha \beta x^2 + \alpha \gamma x + C$$

## FIG. 9

$$y = P(x-q)^2$$

FIG. 10

Blood vessel image search range

FIG. 11

## FIG. 12A

```
┌───────────────────────────┐
│  Hemoglobin concentration │
│    detection processing   │
└───────────────────────────┘
              │
┌───────────────────────────┐
│ Emit 880 nm wavelength light │~S301
└───────────────────────────┘
              │
┌───────────────────────────┐
│       Capture image       │~S302
└───────────────────────────┘
              │
┌───────────────────────────┐
│  Find processing target region │~S303
└───────────────────────────┘
              │
┌───────────────────────────┐
│  Set processing target region │~S304
└───────────────────────────┘
              │
┌───────────────────────────┐
│    Create luminance profile   │~S305
└───────────────────────────┘
              │
┌───────────────────────────┐
│   Create absorption profile,  │~S306
│     determine baseline        │
└───────────────────────────┘
              │
┌───────────────────────────┐
│ Determine blood vessel profile │~S307
└───────────────────────────┘
              │
┌───────────────────────────┐
│     Calculate h1, W1, A1      │~S308
└───────────────────────────┘
              │
            ( 1 )
```

# FIG. 12B

```
        ( 1 )
          |
┌────────────────────────┐
│ Emit 805 nm wavelength light │ ~ S309
└────────────────────────┘
          |
┌────────────────────────┐
│      Capture image      │ ~ S310
└────────────────────────┘
          |
┌────────────────────────┐
│  Set processing target region │ ~ S311
└────────────────────────┘
          |
┌────────────────────────┐
│   Create luminance profile   │ ~ S312
└────────────────────────┘
          |
┌────────────────────────┐
│  Create absorption profile,  │ ~ S313
│     determine baseline       │
└────────────────────────┘
          |
┌────────────────────────┐
│ Determine blood vessel profile │ ~ S314
└────────────────────────┘
          |
┌────────────────────────┐
│    Calculate h2, W2, A2     │ ~ S315
└────────────────────────┘
          |
┌────────────────────────┐
│       Calculate t        │ ~ S316
└────────────────────────┘
          |
┌────────────────────────┐
│   Calculate φ, Hgb, Hct   │ ~ S317
└────────────────────────┘
          |
      ( Return )
```

FIG. 13

FIG. 14

```
        ╭─────────────────────────────╮
        │  Absorption profile creation, │
        │     baseline determination    │
        ╰─────────────────────────────╯
                      │
        ┌─────────────────────────────┐
        │            Set a            │──── S401
        └─────────────────────────────┘
                      │
        ┌─────────────────────────────┐
        │          H←Hmin             │──── S402
        └─────────────────────────────┘
                      │
        ┌─────────────────────────────┐
        │    Clip out luminance profile│──── S403
        └─────────────────────────────┘
                      │
        ┌─────────────────────────────┐
        │  Convert into absorption profile│──── S404
        └─────────────────────────────┘
                      │
        ┌─────────────────────────────┐
        │      Calculate σ and W      │──── S405
        └─────────────────────────────┘
                      │
                    ╱   ╲
           NO     ╱  W=2σ ╲
        ┌───────╱          ╲
        │       ╲          ╱── YES    S406
        │        ╲        ╱
   S407 │          ╲   ╱
  ┌──────────────┐     │
  │    H←H+ΔH    │     │
  └──────────────┘     │
                       │
        ┌─────────────────────────────┐
        │      Determine baseline     │──── S408
        └─────────────────────────────┘
                      │
               ╭─────────────╮
               │    Return   │
               ╰─────────────╯
```

FIG. 15

FIG. 16

FIG. 17

FIG. 18

```
   ╭─────────────────────────────────────────╮
   │ Venous oxygenation index detection processing │
   ╰─────────────────────────────────────────╯
                      │
          ┌───────────────────────┐
          │      Extract Ab2       │──── S501
          └───────────────────────┘
                      │
      ┌───────────────────────────────┐
      │  Emit 660 nm wavelength light   │──── S502
      └───────────────────────────────┘
                      │
          ┌───────────────────────┐
          │      Capture image      │──── S503
          └───────────────────────┘
                      │
      ┌───────────────────────────────┐
      │  Acquire 3rd transmission image  │──── S504
      └───────────────────────────────┘
                      │
          ┌───────────────────────┐
          │      Extract Ab3       │──── S505
          └───────────────────────┘
                      │
          ┌───────────────────────┐
          │      Extract VOI       │──── S506
          └───────────────────────┘
                      │
              ╭───────────────╮
              │    Return     │
              ╰───────────────╯
```

**EP 2 901 928 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2009174766 A1
- US 2002048014 A1
- US 2008306366 A1
- JP 2008067727 A
- JP 2000262496 A